(19) Europäisches Patentamt
European Patent Office
Office européen des brevets

(11) **EP 1 916 308 A1**

(12) **EUROPEAN PATENT APPLICATION**

(43) Date of publication:
**30.04.2008 Bulletin 2008/18**

(51) Int Cl.:
***C12P 13/08*** (2006.01)

(21) Application number: **06022431.8**

(22) Date of filing: **26.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **DSM IP Assets B.V.**
**6411 TE  Heerlen (NL)**

(72) Inventors:
• **Flores-Candia, Juana-Lucia**
**4126 Bettingen (CH)**
• **Lorenz, Eric**
**89077 Ulm (DE)**

(74) Representative: **Keller, Günter et al**
**Lederer & Keller**
**Patentanwälte**
**Prinzregentenstrasse 16**
**80538 München (DE)**

(54) **Use of vitamins in fermentation processes for the production of amino acids**

(57)    The present invention refers to the use of vitamins in a fermentation process for the production of e.g. amino acids or ethanol, wherein at least 4 vitamins selected from the group consisting of: thiamine, cobalamine, riboflavine, niacinamide, pantothenic acid, biotin, ascorbic acid, retinol, procalciol, tocopherol, folic acid and pyridoxamine are used, and wherein the use of these vitamins enhances the bacterial growth rate ($\mu$) at least by 50 % and the product concentration [g/L] at least by 10 % compared to a reference fermentation process, performed without adding vitamins.

**EP 1 916 308 A1**

**Description**

The present invention refers to the use of vitamins in a fermentation process, wherein at least 4 vitamins selected from the group consisting of: thiamine, cobalamine, riboflavine, niacinamide, pantothenic acid, biotin, ascorbic acid, retinol, procalciol, tocopherol, folic acid and pyridoxamine are used, and wherein the use of these vitamins enhances the bacterial growth rate ($\mu$) at least by 50 % and the product concentration [g/L] at least by 10 % compared to a reference fermentation process, performed without adding vitamins.

The most important amino acids produced by fermentation reactions are L-glutamic acid and L-lysine. Both are produced in fermentations with *Corynebacterium glutamicum* or the closely related *Brevibacterium lactofermentum* or *B. flavum.* These microorganisms are biotin-auxotrophic.

The industrial production of amino acids is performed in submerse-processes, using batch, fed-batch or continuous fermentation processes. Important parameters, which have to be controlled during the fermentation process on a high scale are: pH, aeration, feeding rate and process temperature.

*C. glutamicum* can be used as a microorganism for the production of L-glutamic acid and L-lysine, wherein a lot of mutants of this bacterium have been developed to increase the product yield. *C. glutamicum* is auxotrophic in relation to biotin, which has to be provided with the medium (Nakayama et al., J. Gen. Appl. Microbiol. 1961, 7, 145 - 154). Furthermore, glucose is used as a C-source for the microorganism. However, industrial media comprising vitamins for the production of L-lysine are unknown.

Early studies showed that glutamate excretion is triggered by a biotin-limitation of the bacteria (2 - 3 $\mu$g/L), whereas at higher biotin concentrations (> 5 $\mu$g/L) lysine is released to the medium. It is also known that glutamate excretion can be achieved at higher biotin concentrations, if surfactants are added (Takinami et al., Biol. Chem. 1965, 29, 351 - 359).

The most effective approach to influence the product formation of a microorganism in a fermentation process is to optimize the fermentation medium. The medium for a fermentation process should comprise all the elements which are required for the growth of the microorganism and for the product formation. Media for fermentation processes can be divided into the group of defined or complex media. Defined media only contain exactly defined ingredients in known concentrations. Contrary to that, complex media do also comprise one or more unknown organic ingredients or organic ingredients in an unknown amount.

For technical fermentations, molasses or starch hydrolysates are used as C-sources and ammonium sulfate as N-source, respectively. Furthermore, it is known that vitamins like biotin can be added to C. *glutamicum* fermentations in their pure form. However, it remains unclear, whether the use of defined media in the industrial fermentation processes results in a higher product conversion efficiency or an enhanced bacterial growth rate.

Up to now, complex media have been preferred for industrial applications, due to their lower price and the high amounts which are needed. Complex media for industrial scale applications are for example soy flour, molasses or corn steep liquor. However, problems are related with the use of these complex media. For example when using molasses, problems arise due to the fluctuations in the concentration of the ingredients/ nutrients, especially the vitamins, depending on the season of the year. Furthermore, the amounts of desired nutrients per weight in molasses declines, because the processes which provide molasses as a waste or byproduct are continuously improved. Accordingly, an cost effective use of defined media in industrial fermentation processes would allow to avoid the fluctuations of the ingredients/ nutrients and would provide a better control of the fermentation process.

In order to overcome the drawbacks of complex media for the use in industrial fermentation processes, vitamin compositions have been prepared and their effect on the fermentation performance has been analysed. It has been found that the use of vitamins according to the present invention allows to reduce the operational time of the fermentation process due to an enhanced bacterial growth rate in the first batch-phase. In case of lysine production, an earlier limitation of the culture broth by threonine is caused thereby, which results in an earlier lysine production.

Accordingly, the use of vitamins according to the present invention results in a faster biomass development, wherein the final biomass concentration is reached in a shorter time. For example the use of vitamin composition 1 (example 12) allows to achieve a specific bacterial growth rate in the batch part of the process which is twice as high compared to the fermentation process without the composition.

Furthermore, the operational time of the fermentation process, until a defined amount of glucose is completely consumed, has been reduced by about 22% up to about 43%. To reduce the operational time of a fermentation process results in a cost decrease for the process. The operational time of the fermentation process was also reduced compared to known industrial fermentation processes using complex media. This results in a desired cost reduction in industrial applications. When carrying out processes according to the present invention, it has been found that a cost reduction can be achieved, which allows a cheaper industrial fermentation process with pure vitamins than with complex media.

The use of the vitamin composition 2 (example 13) enhances the bacterial growth rate in the batch part of the fermentation process by 50 %, wherein the fermentation time could be reduced by 6 hours to a total of 36 hours. The bacterial growth rate could also be increased and the operational time of the fermentation process could be reduced, compared to industrial fermentation processes using complex media.

The use of vitamins according to the present invention comprises to use several vitamins of the B-group. It has been found that it is most important to use at least 4, preferably 5, vitamins from the B-group in order to achieve a very high bacterial growth rate. It has furthermore been found that using less than 4, preferably 5 vitamins does not allow to improve the fermentation process. For example, it has been found that the use of niacinamide alone inhibits the bacterial growth rate (compare example 14). In this example, no lysine formation was observed. Contrary to this, the use of a high amount of niacinamide in combination with the other B-vitamins according to the present invention allows to improve the fermentation performance. This is discussed at example 14.

The use of the vitamin compositions according to the present invention in example resulted in an increased product concentration (amino acid) to about 38 g L$^{-1}$ which is 14% higher compared to a fermentation process without the vitamin composition. This increase in the product concentration allows to improve the cost efficiency of industrial fermentation processes.

Furthermore, the use of the vitamins according to the present invention increased the resistance of the microorganisms against ethanol in a fermentation process for the production of bioethanol.

The present invention therefore refers to the use of vitamins in a fermentation process for the production of any compounds, which can be produced by microorganisms, preferably amino acids or ethanol, further preferred amino acids, and even further preferred lysine, characterized in that at least 4 vitamins, further preferred 5 vitamins, even further preferred 7 vitamins and most preferred 8 vitamins, selected from the group consisting of: thiamine, cobalamine, riboflavine, niacinamide, pantothenic acid, biotin, ascorbic acid, retinol, procalciol, tocopherol, folic acid and pyridoxamine are used, and wherein the use of these vitamins enhances the bacterial growth rate at least by 50 %, further preferred 100 %, and the product concentration at least by 10 %, further preferred 20 %, compared to a reference fermentation process, performed without adding vitamins.

Furthermore, the improved bacterial growth rate ($\mu$), the enhanced yield and the reduction of the operational time allows to increase the fermentation throughput by at least 34 %. This is an important aspect for industrial production.

In a preferred embodiment of the invention, at least 5 vitamins, even further preferred at least 6 vitamins, selected from the group consisting of: thiamine, riboflavine, niacinamide, pantothenic acid, biotin and pyridoxamine, are used in the fermentation process.

It is also preferred to use the vitamins thiamine, riboflavine, nicacinamide, pantothenic acid, biotin and cobalamine.

In a preferred embodiment, the volumetric productivity is increased by at least 30 %, further preferred at least 40 % compared to a reference process without using the vitamins according to the present invention.

In another preferred embodiment, ascorbic acid is used as one of the vitamins.

It is furthermore preferred that the microorganism for the fermentation process is *Corynebacterium glutamicum* (for amino acid production) and *Saccharomyces cerevisiae* (for ethanol production).

It is also preferred that lysine is produced in the fermentation process.

In a preferred embodiment of the invention, the vitamins allow to reduce the duration of the lag phase at least by 15 % compared to a reference fermentation process, performed without adding vitamins.

In an even further preferred embodiment, thiamine, riboflavine, niacinamide, panthothenic acid and biotin are used in the fermentation process, wherein the amount of all vitamins, added in pure form is at least 19 mg/L, further preferred 19-94 mg/L and most preferred 50-94 mg/L in the fermentation medium.

In a preferred embodiment of the invention, a surfactant is used in addition to the vitamins, preferably polyethylene glycol 2000 is used as a surfactant.

The microorganisms, which can be used for the fermentation according to the present invention can be any microorganisms, suitable for the production of the desired product. Microorganisms of the species *Corynebacterium glutamicum, Brevibacterium lactofermentum, B. flavum, Saccharomyces cerevisiae* (for ethanol production), *Saccharomyces uvarum* (for ethanol production), *Candida utilis, Kluyveromyces* and *Zymomonas mobilis* can preferably be used. Further preferred for the production is *Saccharomyces cerevisiae.* Even further preferred for the amino acid production is the use of *Corynebacterium glutamicum* and most preferable is the use of *Corynebacterium glutamicum* with the deposition number ATCC (American Type Culture Collection) 21543 in fermentation processes according to the present invention.

Products of the fermentation process can be all compounds, which can be produced by microorganisms. Preferably the compound is selected from the group consisting of: amino acids, ethanol, citric acid, lactic acid, antibiotics, steroids, therapeutics and enzymes. Further preferred, the compound is an amino acid or ethanol, even further preferred amino acids.

Amino acids which can be produced by the fermentation according to the present invention can be all amino acids, which can be produced by microorganisms. Preferably, the amino acids to be produced are L-glutamic acid and L-lysine (in the following also "lysine"), further preferred L-lysine.

The vitamins which can be used for the fermentation processes according to the present invention are: thiamine, cobalamine, riboflavine, niacinamide, pantothenic acid, biotin and pyridoxamine. Preferably 4 vitamins, further preferred 5 vitamins and even further preferred 6 vitamins, are selected from this group of vitamins. It is most preferred that biotin is used as one of the vitamins.

The term "use of vitamins according to the present invention" refers to the addition of pure vitamins or a premixed vitamin composition to the fermentation medium and/ or the feeding solution. However, the medium, preferably the preculture medium, can in addition already contain complex media like for example yeast extract and furthermore vitamins on which the microorganism is auxotrophic (e.g. biotin). In addition, ascorbinic acid can be used together with the other vitamins.

The term "vitamin composition" refers to a composition containing all the vitamins, which are added to the fermentation process in pure form. The vitamin composition can also contain carrier compounds.

Furthermore, the amount of vitamins used for the fermentation process, can be determined by a person skilled in the art. Preferably the end concentration of all the vitamins (without carrier or other additional compounds) according to the present invention is at least 19 mg/L, further preferred between $19 \, g \, L^{-1}$ and $94 \, g \, L^{-1}$ in the fermentation medium.

Alternatively, the vitamins can be independently selected and used in the amounts [mg/L] in the fermentation medium as specified in Table 1:

**Table 1: Amounts of vitamins**

| Vitamin | Amount [mg/L] | Preferred Amount [mg/L] | Further preferred amount [mg/L] |
|---|---|---|---|
| Thiamine | 2 - 50 | 10 - 50 | 20 - 50 |
| Riboflavine | 1 - 100 | 20 - 100 | 25 - 100 |
| Cobalamine | 1 - 10 | 2 - 10 | 3 - 10 |
| Niacinamide | 1 - 100 | 10 - 100 | 15 - 100 |
| Pantothenic acid | 2 - 50 | 5 - 50 | 10 - 50 |
| Biotin | 2 - 40 | 4 - 40 | 10 - 40 |
| Pyridoxamine | 2 - 50 | 5 - 50 | 10 - 50 |
| Folic acid | 0,05 - 5 | 0,1 - 5 | 1 - 5 |
| Ascorbinic acid | 1 - 500 | 150 - 500 | 250 - 500 |
| Retinol | 0.1 - 50 | 1- 50 | 10 - 50 |
| Procalciol | 1 - 10 | 2 - 10 | 5 - 10 |
| Tocopherol | 1 - 80 | 10-80 | 30 - 80 |

The microorganisms of the present invention are cultured under controlled aeration. The term "controlled aeration" includes sufficient aeration (e.g., oxygen) to result in the production of the desired product. It is preferred that air is sparked through the fermentation containment. The dissolved oxygen partial pressure ($pO_2$) can be measured during the fermentation process, e.g. with polarographic oxygen probes or using the DCU (digital control unit). The agitation speed and aeration can be used to control the $pO_2$, wherein a stepwise increase of the agitation speed might be necessary to control the $pO_2$ at preferred values of $\geq 40\%$, more preferred of $\geq 65\%$.

In addition it is preferred to use the vitamins in industrial scale fermentation processes. The preferred working volume in these fermentation processes is 100000 L, further preferred, 150000 L and most preferred 200000 L.

In addition, the fermentation is preferably performed at a pH-value of 7 and a temperature of 30° C. The pH-value can e.g. be controlled using the DCU (micro DCU-300, obtained from B. Braun Biotec International / Satorius) and the temperature can be controlled using an external thermostate. Preferably, the adjustment of the pH- value is performed using $NH_4OH_{aq}$ and $3M \, H_2SO_4$.

The fermentation process using vitamins according to the present invention can be performed using any known type of fermentation e.g. batch, fed-batch or continuous fermentation processes.

In addition, a surfactant, preferably polyethylene glycol 2000, can be used as means for antiforming and can be added in addition to the vitamins if necessary.

Appropriate culture medium (or production medium) can be chosen by a person skilled in the art. An exemplary culture medium is given in the examples.

The feeding rate can be determined using the equation as given in example 11. The feeding solution used for the fermentation process comprises glucose preferably in an amount of 300-800 g/L, further preferred 500-800 g/L. The feeding is performed at a continuous feeding rate.

The use of vitamins in a fermentation process according to the present invention increases the bacterial growth rate ($\mu$). The bacterial growth rate [$h^{-1}$] is increased by at least 50 % compared to the bacterial growth rate in a reference fermentation, preferably the bacterial growth rate is increased by at least 60 %. Reference fermentations are carried out using the same fermentation medium, the same feeding solution and the same feeding rate, but without adding the vitamins according to the present invention. However, if biotin auxotrophic microorganisms are used, the fermentation medium or the feeding solution can already contain biotin. Furthermore the preculture medium can already contain complex media like yeast extract (compare example 2). The bacterial growth rate ($\mu$) can be determined by measuring the dry biomass concentration or by using any technique known in the art. The dry biomass concentration can be determined as shown in example 6 or by any method known in the art. However, the methods for determining the bacterial growth rate in the reference fermentation and in the fermentation using vitamins according to the present invention have to be the same.

The use of vitamins in a fermentation process according to the present invention increases the average overall product concentration [g/L] over the whole fermentation process by at least 10 % compared to the average overall product concentration [g/L] in a reference fermentation. Reference fermentations are carried out as indicated above. The product concentration can be determined by measuring the amount of amino acids which is produced. The determination of the product can be performed by using any technique known in the art. If lysine is produced, e.g. the method as described in example 8 can be used. However, the methods for determining the product concentration in the reference fermentation and in the fermentation using vitamins according to the present invention have to be the same.

The use of vitamins in a fermentation process according to the present allows to reduce the operational time of the fermentation process. This results in a desired cost reduction in industrial applications. The operational time of the fermentation process until a defined amount of glucose is completely consumed, can been reduced by at least 15 %, further preferred by at least 20 %, even further preferred by at least 25 % and most preferred by at least 30 % compared to a reference fermentation process as described above.

Additionally the present invention includes a step of recovering the desired compound (e.g., pantothenate). The term "recovering" a desired compound includes extracting, harvesting, isolating or purifying the compound from the culture medium.

Recovering the compound can be performed according to any conventional isolation or purification methodology known in the art including, but not limited to, treatment with a conventional resin (e.g., anion or cation exchange resin, non-ionic adsorption resin, etc.), treatment with a conventional adsorbent (e.g., activated charcoal, silicic acid, silica gel, cellulose, alumina, etc.), alteration of pH, solvent extraction (e.g., with a conventional solvent such as an alcohol, ethyl acetate, hexane and the like), dialysis, filtration, concentration, crystallization, recrystallization, pH adjustment, lyophilization and the like. For example, a compound can be recovered from culture medium by first removing the microorganisms from the culture. Medium are then passed through or over a cation exchange resin to remove cations and then through or over an anion exchange resin to remove inorganic anions and organic acids having stronger acidities than the compound of interest.

Preferably, a desired compound of the present invention is "extracted", "isolated" or "purified" such that the resulting preparation is substantially free of other medium components (e.g., free of medium components and/or fermentation byproducts). The language "substantially free of other medium components" includes preparations of the desired compound in which the compound is separated from medium components or fermentation byproducts of the culture from which it is produced. In one embodiment, the preparation has greater than about 80% (by dry weight) of the desired compound (e.g., less than about 20% of other medium components or fermentation byproducts), more preferably greater than about 90% of the desired compound (e.g., less than about 10% of other medium components or fermentation byproducts), still more preferably greater than about 95% of the desired compound (e.g., less than about 5% of other medium components or fermentation byproducts), and most preferably greater than about 98-99% desired compound (e.g., less than about 1-2% other medium components or fermentation byproducts). When the desired compound has been derivatized to a salt, the compound is preferably further free of chemical contaminants associated with the formation of the salt. When the desired compound has been derivatized to an alcohol, the compound is preferably further free of chemical contaminants associated with the formation of the alcohol.

In an alternative embodiment, the desired compound is not purified from the fermentation broth of the microorganism, for example, when the microorganism is biologically non-hazardous (e.g., safe). For example, the entire culture (or culture supernatant) can be used as a source of product (e.g., crude product). In one embodiment, the culture (or culture supernatant) is used without modification. In another embodiment, the culture (or culture supernatant) is concentrated. In yet another embodiment, the culture (or culture supernatant) is dried or lyophilized.

**Description of the Figures:**

**Figure 1:** Shows the culturing of *C. glutamicum* ATCC 21543 in the reference fermentations, referring to the bacterial growth rate.

**Figure 2:** Shows the culturing of *C. glutamicum* ATCC 21543 in the reference fermentations, referring to the lysine production.

**Figure 3**: Shows the culturing of *C. glutamicum* ATCC 21543 in the reference fermentations, referring to the threonine concentration.

**Figure 4:** Shows the culturing of *C. glutamicum* ATCC 21543 using vitamin composition 1, referring bacterial growth rate.

**Figure 5:** Shows the culturing of *C. glutamicum* ATCC 21543 using vitamin composition 1, referring to the lysine production.

**Figure 6:** Shows the culturing of *C. glutamicum* ATCC 21543 using vitamin composition 1, referring to the threonine concentration.

**Figure 7:** Shows the culturing of *C. glutamicum* ATCC 21543 using vitamin composition 2, referring to the bacterial growth rate.

**Figure 8:** Shows the culturing of *C. glutamicum* ATCC 21543 using vitamin composition 1, referring to the lysine production.

**Figure 9:** Shows the culturing of *C. glutamicum* ATCC 21543 using vitamin composition 1, referring to the threonine concentration.

**Figure 10:** Shows the culturing of *C. glutamicum* ATCC 21543 using only niacinamide, referring to the bacterial growth rate.

**Figure 11:** Shows the culturing of *C. glutamicum* ATCC 21543 using only niacinamide, referring to the threonine concentration.

**Example 1- Stock solutions and culture media**

*1 M potassium phosphate buffer*

53.56 g $K_2HPO_4$ and 26.20 g $KH_2PO_4$ have been dissolved in 500 mL $dH_2O$ and filled up to 1000 mL, wherein the pH was 7.

*500 mM sodium borate buffer*

6.183 g boric acid was dissolved in 100 mL $dH_2O$. The pH value was adjusted to 8 using 3N NaOH solution. Subsequently, 200 mL $dH_2O$ were added.

*50 mM sodium acetate buffer*

2.875 glacial acetic acid were mixed with 500 mL $dH_2O$ and the pH value was adjusted to 4.25 by using 3N NaOH solution. Subsequently, $dH_2O$ was added until a volume of 1000 mL was reached.

*10 mM Fmoc-Cl-acetonitrile solution*

7.8 mg Fmoc-Cl were mixed with 3 mL of acetonitrile.

*20 mM 1-Adamantylamine-methanol solution*

9.1 mg adamantylamine were mixed with 3 mL methanol.

*100 mM L-lysine stock solution*

0.146 g L-lysine were dissolved in 5 mL $dH_2O$ and $dH_2O$ was added to a final volume of 10 mL.

*9 % physiological sodium chloride solution*

90 g sodium chloride were dissolved in 500 mL $dH_2O$ and subsequently, $dH_2O$ was added to reach a final volume of 1000 mL.

*100 mM L-threonine stock solution*

0.119 g L-threonine were dissolved in 5 mL $dH_2O$ and subsequently, $dH_2O$ was added to reach a final volume of 10 mL.

*100 mM L-methionine stock solution*

0.149 g L-methionine were dissolved in 5 mL $dH_2O$ and subsequently, $dH_2O$ was added to reach a final volume of 10 mL.

*100 mM L-leucine stock solution*

0.131 g L-leucine was dissolved in 5 mL $dH_2O$ and subsequently, $dH_2O$ was added to reach a final volume of 10 mL.

**Example 2 - Sterilization and media formulation**

The agar preculture medium (Table 2) and the medium for the first preculture (Table 3) were autoclaved in the following manner: Yeast extract, tryptophane, NaCl and Agarose were dissolved in 250 mL $dH_2O$ and subsequently, $dH_2O$ was added to reach a final volume of 500 mL and separately autoclaved. Glucose was dissolved in 250 mL $dH_2O$ and subsequently, $dH_2O$ was added to reach a final volume of 500 mL, this solution was separately autoclaved. The two media solutions were than mixed under the sterile workbench.

**Table 2 Ingredients of the agar preculture medium (**Kiss et al., Biotechnol. Prog. 1991, 7,501-508**).**

| Agar preculture medium | | |
|---|---|---|
| **Compound** | **Concentration** | |
| Yeast extract | 5 | g/L |
| Tryptone | 10 | g/L |
| NaCl | 10 | g/L |
| Glucose | 5 | g/L |
| Agarose | 18 | g/L |

**Table 3 Preculture medium I (**Kiss et al., Biotechnol. Prog. 1991, 7, 501-508**).**

| Preculture medium I | | |
|---|---|---|
| **Compound** | **Concentration** | |
| Yeast extract | 5 | g/L |
| Tryptone | 10 | g/L |
| NaCl | 10 | g/L |
| Glucose | 5 | g/L |

The ingredients of the medium preculture II (Table 4) were dissolved in 500 mL $dH_2O$ and subsequently, $dH_2O$

was added to reach a final volume of 1 L. The solution was than sterile filtrated through a 0.22 $\mu$m filter. In order to avoid a clouding of the solution or the precipitation of compounds, $MgSO_4$, $FeSO_4$ and $CaCl_2$ were at first dissolved in $dH_2O$ and subsequently the media was added. The fermentation media and the feeding solution were treated in the same manner.

The fermentation medium was sterilized in two parts. The glucose was dissolved in 1 L $dH_2O$ and the pH value was adjusted to 2 - 3 by using 3 M $H_2SO_4$. Subsequently, $dH_2O$ was added to reach a final volume of 1.5 L and then the fermenter was autoclaved. The remaining ingredients of the medium were then dissolved in 1.5 L $dH_2O$ and subsequently $dH_2O$ was added to reach a final volume of 2 L. The solution was then sterile filtrated through a 0.22 $\mu$m filter.

**Table 4 : Preculture medium II and fermentation medium (**Kiss et al., Biotechnol. Prog. 1991, 7, 501-508**).**

| Preculture medium II and fermentation medium | | |
|---|---|---|
| **Compound** | **Concentration** | |
| Glucose | 50 | g/L |
| Citrate | 0,5 | g/L |
| $CaCl_2$ | 1 | g/L |
| $MgSO_4*7H_2O$ | 600 | mg/L |
| $FeSO_4*7H_2O$ | 50 | mg/L |
| NaCl | 2 | g/L |
| Na-EDTA | 75 | mg/L |
| Solution of trace elements | 20 | mUL |
| $KH_2PO_4$ | 2 | g/L |
| $K_2HPO_4$ | 4 | g/L |
| L-Threonine | 733 | mg/L |
| L-Methionine | 600 | mg/L |
| L-Leucine | 1500 | mg/L |
| $(NH_4)_2SO_4$ | 40 | g/L |
| Biotin | 1 | mg/L |

**Table 5: Feeding solution (**Kiss et al., Biotechnol. Prog. 1991, 7, 501-508**).**

| Feeding solution | | |
|---|---|---|
| **Compound** | **Concentration** | |
| Glucose | 400 | g/L |
| Citrate | 0,5 | g/L |
| $CaCl_2$ | 1 | g/L |
| $MgSO_4*7H_2O$ | 600 | mg/L |
| $FeS04*7H_2O$ | 50 | mg/L |
| NaCl | 2 | g/L |
| Na-EDTA | 75 | mg/L |
| Solution of trace elements | 20 | mUL |
| $KH_2PO_4$ | 2 | g/L |
| $K_2HPO_4$ | 4 | g/L |
| L-Threonine | 733 | mg/L |

(continued)

| Feeding solution | | |
|---|---|---|
| Compound | Concentration | |
| L-Methionine | 600 | mg/L |
| L-Leucine | 1500 | mg/L |
| $(NH_4)_2SO_4$ | 40 | g/L |
| Biotin | 1 | mg/L |

Table 6: Solution of trace elements (Kiss et al., Biotechnol. Prog. 1991, 7, 501-508).

| Solution of trace elements | | |
|---|---|---|
| Compound | Concentration | |
| $MnSO_4$ | 200 | mg/L |
| $Na_2B4O_7*10H_2O$ | 20 | mg/L |
| $(NH_4)_6Mo_7O_{24}*4H_2O$ | 10 | mg/L |
| $FeCl_3*6H_2O$ | 200 | mg/L |
| $ZnSO_4*7H_2O$ | 50 | mg/L |
| $CuCl_2*2H_2O$ | 20 | mg/L |

The ingredients for the feeding solution (Table 5) were dissolved in 1.5 L $dH_2O$ and subsequently $dH_2O$ was added to reach a final volume of 2 L. The solution was then sterile filtrated through a 0.22 $\mu$m filter.

The trace elements (Table 6) were dissolved in 0.5 L $dH_2O$ and adjusted to a pH value of 2 by using 3 M $H_2SO_4$. Subsequently, $dH_2O$ was added to reach a final volume of 1 L. The solution was then sterile filtrated through a 0.22 $\mu$m filter.

**Example 3 - Vitamin compositions**

The vitamin compositions for the fermentation processes, composition 1 and composition 2, are shown in Table 7.

Table 7: Ingredients of the vitamin composition 1 and vitamin composition 2.

| Vitamins | Vitamin composition 1 | Vitamin composition 2 |
|---|---|---|
| | [mg/g] | [mg/g] |
| Thiamine** | 8,05 | 76,00 |
| Riboflavine** | 8,80 | 455,00 |
| Niacinamide** | 94,50 | 303,00 |
| Folic acid** | 1,25 | |
| Pantothenic acid** | 37,50 | 151,00 |
| Biotin | 0,94 | 15,00 |
| Cobalamine** | 6,50E-04 | |
| Ascorbinic acid | 330,00 | |
| Pyridoxamine** | 11,00 | |
| Retinol* | 5,3987 | |
| Pro-calciol* | 3,25E-02 | |
| Tocopherole* | 55,00 | |

(continued)

| Vitamins | Vitamin composition 1 | Vitamin composition 2 |
|---|---|---|
| | [mg/g] | [mg/g] |
| Carrier | 447,53 | |
| *fat soluble vitamins; ** B-group vitamins | | |

The final concentration of the vitamin composition in the fermentation medium was 1g L$^{-1}$. In order to reach that concentration in the fermentation medium, the required 4 g of composition 1 were dissolved in 500 mL dH$_2$O. Subsequently, the mixture was centrifugated and separated from the solids, which have not been dissolved. The centrifugation was performed by 63.000 x g and 30 minutes. The supernatant was decanted, mixed with the fermentation media and sterile filtrated. The volume of the vitamin solution was considered when dissolving the other medium ingredients, which means that less dH$_2$O was used when preparing the fermentation medium.

The final concentration of composition 2 in the fermenter was 66 mg . L$^{-1}$. In order to reach that concentration, 264 mg of composition 2 were dissolved in 2 L fermentation medium. The resulting mixture was then sterile filtrated. By doing so, 20 % of the used riboflavine, comprised in composition 2, was separated from the fermentation medium. Furthermore, 132 mg of composition 2 were used for the 2 L feeding solution, wherein composition 2 was completely dissolved. However, the feeding solution was sterile filtrated after dissolution of all ingredients.

The final concentration of niacinamide, used in the nicacinamide fermentation processes, was 94 mg L$^{-1}$ in the fermenter. In order to obtain this final concentration, 376 mg niacinamide were dissolved in 2 L fermentation medium and 188 mg were added to the 2 L feeding solution.

## Example 4 - Handling of the microorganism

3 mL of the liquid culture were incubated over night at 30° C in a 15 mL tube. The cells were then centrifugated at 2750 x g for 10 minutes. The supernatant was discharged and the cell pellet was suspended in 1.8 mL of fresh preculture medium I. Then, 400 μL glycerin (99 % grade) and 600 μL of the cell suspension were added into a 2 mL cryotube. The tube was vortexed and slowly cooled with ice and then the tube was frozen at -80° C.

## Example 5 - Determination of the glucose concentration

The determination of the glucose concentration was performed using the kit obtained from Roche Diagnostics (Glucose Schnelltest-Kit) and was performed according to the instructions. The data provided in the examples are average values obtained from a set of fermentation processes. The glucose concentration was determined by using the following equation:

$$c = \frac{V * Mr * \Delta E}{\varepsilon * d * v * 1000}$$

**Equation 1: Calculation of the glucose concentration. c=glucose concentration [g/L]; V= test volume [mL]; v=sample volume [mL]; d=thickness of the layer [cm]; $\varepsilon$=extinction coefficient [l/mmol*cm]; Mr= molecular weight of glucose [g/mol]; $\Delta E$ is the differential of absorbance.**

## Example 6 - determination of the dry biomass concentration

In order to determine the dry biomass concentration, a 15 mL Falcon-tube was stored at 70° C for 24 hours in a drying oven and then cooled down in a desiccator. The weight of the tube was then determined using a precision balance. A 10 mL sample was then taken from the bioreactor and subsequently centrifugated at 4° C, 2750 x g and 15 min. The supernatant was then sterile filtrated in Eppendorf-tubes and stored at -20° C for further analysis. The sediment was then washed with a physiological sodium chloride solution and once more centrifugated for 15 minutes at 2750 x g. The supernatant was decanted and the tube was stored for 24 hours at 70° C in the drying oven. Afterwards, the tube was stored in a desiccator for 12 hours in order to cool down. The tubes were then again weight out with a precision balance. The differences of the masses resulted in the value for the dry biomass concentration.

**Example 7 - Determination of the optical density at 600 nm (OD$_{600}$)**

A sample was taken from the bioreactor in order to photometrically determine (at 600 nm) the 00$_{600}$. The photometers used in the present invention were "Photometer lambda 25" (from Perkin Elmer) and "Photometer Ultraspec 100 pro" (from Ambersham Biosciences). The measured extinction of the photometer should be in a range from 0.1 - 0.4. If the OD$_{600}$ was outside the range of linearity, a physiological sodium chloride solution was used in order to dissolute the solution, which was then again measured.

**Example 8 - Determination of the amino acids**

In order to determine the amino acids, the supernatant, which was obtained by centrifugation of the sample, was filtrated using a 0.22 μm filter with a PVDF-membrane. The filtrate was then used for analysis.

The derivatisation of the amino acids was performed in a alkaline environment, using 100 mM sodium borate buffer. The derivatisation of the amino acids was performed using 9-fluorenylmethyl-chloroformate (Fmoc-Cl), according to the procedure of Einarsson et al. (Anal. The. 1986, 58, 1638 - 1643).

Fmoc-Cl was added in excess, wherein the excess of Fmoc-Cl, which did not react with the amino acids, was quenched using 1-adamantylamine. Fmoc-Cl was dissolved in acetonitrile and the 1-adamantylamine in methanol. The derivatisation procedure was performed as follows:

First of all, the sample was dissolved with sodium borate buffer in order to reach an amino acid concentration in the range of the calibration line (0.2 - 2 mM). 150 μL of this dissolved sample was then mixed with 150 μL Fmoc-Cl at room temperature. After 2 minutes of incubation, 150 μL 1-adamantylamine were added to the sample and incubated for 5 minutes at room temperature. After the incubation, 350 μL of the sample were filled in a test tube, which was closed subsequently. The analysis of the sample was then performed using HPLC (high performance liquid chromatography). The stationary face was a Eurobond C-18 column (obtained from Bischoff). The mobile phase was acetonitrile and sodium acetate buffer. The flow rate in the HPLC-procedure was 1.5 mL/min.

In order to separate the amino acids, 2 different programs were used. The resulting values are given in percent by volume. The program for analysis of L-threonine and L-leucine was started at 30 % acetonitrile and 70 % sodium acetate buffer for 0 - 5 minutes. Afterwards, over a period of time of 11 minutes, 50 % acetonitrile and 50 % sodium acetate buffer were used. Within 2 minutes, acetonitrile was increased to 100 %, wherein this level was kept for 3 minutes. Subsequently, the acetonitrile was then decreased to 30 % and the sodium acetate buffer increased to 70 %, wherein these levels were kept for 3 minutes until the end of the program. The overall operational time of this program for analysis of the above indicated amino acids L-threonine and L-leucine was 24 minutes.

The program for analysis of L-leucine was started at 60 % acetonitrile and 40 % sodium acetate buffer for 4 minutes. Subsequently, until 7 minutes, the acetonitrile level was increased to 100 % and the sodium acetate buffer was decreased to 0 %, wherein these levels were kept for 2 minutes. Afterwards, the acetonitrile level was decreased to 60 % and the sodium acetate buffer was increased to 40 %, wherein these levels were kept for further 2 minutes. The overall operational time of the HPLC program for the analysis of the amino acid L-leucine was 12 minutes.

**Example 9 - Quantitative analysis of riboflavine**

In order to determine riboflavine in a quantitative manner, firstly an absorption spectra of the pure substance was measured. The absorption maximum of riboflavine was then determined at 267 nm.

A calibration was performed in order to determine riboflavine in a quantitative manner. When analyzing the fermentation medium, the fermentation medium was prepared and sterile filtrated through a 0.22 μm nitrocellulose filter. The filtrate was stored and the filter was dried for 48 hours in a desiccator. The dry filtrate was added to a sodium phosphate buffer at pH 7 and shaken for 1 hour at room temperature. By doing so, the riboflavine from the filter was dissolved in the liquid phase and could be determined.

**Example 10 - Averaging the sets of data**

If the sets of data of different fermentation reactions were taken together, a linear interpolation and calculation of average values were performed. As the samples were taken at different time points in the fermentation procedure, a linear interpolation of the 2 adjacent data points allowed the calculation of a data point for the desired point of time. Accordingly, the data points of different fermentation reactions were assigned to specific points of time and allowed to calculate the average value. Furthermore, a confident interval for the probabilities for an error of 10 % were calculated using the 3 data points of a time point, in order to determine, whether the differences between the reference fermentations and the fermentations with the compositions according to the invention were significant.

**Example 11 - Fermentation**

*Fermentation parameter*

The total volume of the glass fermenter tank (from B. Braun Biotec International) was 10 L and the working volume was set at a maximum of 6 L. Fermentation was performed at a pH-value of 7 and a temperature of 30° C. The pH-value was controlled using the DCU (micro DCU-300, obtained from B. Braun Biotec International / Satorius) and the temperature was controlled using an external thermostate. The adjustment of the pH-value was performed using $NH_4OH_{aq}$ and $3MH_2SO_4$. Polyethylene glycol 2000 was used as means for antiforming and was added when necessary. The $pO_2$ was controlled at 65 % using the DCU and adjusted by increasing the agitation speed and then by increasing the aeration.

*Sterilization of the fermenter and calibration of the electrodes*

Before the fermenter was sterilized, the pH-electrodes had to be calibrated and the $pO_2$-probe was filled with electrolyte solution. A 2-point-calibration using solutions at pH 4 and pH 7, was performed. Calibration was performed according to the instructions of the DCU. The fermenter was sterilized at 120° for 21 minutes. After the sterilization of the fermenter, the $pO_2$-probe was connected to the DCU and polarized for 6 hours. After the polarization, the probe was calibrated. The calibration was performed according to the instructions of the DCU.

***Precultures***

In order to prepare the agar cultures, 10 $\mu$L cell suspension was taken from the glycerin culture (described above) and plated. The agar plate was then incubated for 24 hours at 30° C in the incubator. The obtained colonies were used to inoculate 50 mL of the preculture medium 1. The incubation was performed in a 500 mL Erlenmeyer flask (comprising baffles) at 30° C and 150 upm in a shaker for 8 - 10 hours up to a OD of 6 - 8. After the incubation, the preculture medium 1 was transferred into 500 mL of preculture medium II. The incubation was performed in a 3 L Erlenmeyer flask (with baffles) at 30° C and 100 upm in a shaker for 8 - 10 hours up to a OD of 15 - 18.

***Inoculation of the fermenter***

After incubation of the preculture medium II, culture was transferred into a 1 L Schott flask. The Schott flask was provided with a 0.45 $\mu$m filter for equalization of the pressure. Furthermore the Schott flask comprised means for inoculation, consisting in a silicon tube, a screw cab, a head piece with two olives and a injection needle. The preculture medium II was transferred into the fermenter using a pump.

***Feeding***

The calculation of the exponential feeding profile was performed using the following equation:

$$F = \frac{\mu}{Y_{X/S}} * V_0 * \frac{X_0}{C_{Feed}} * e^{\mu * t}$$

**Equation 1: Calculation of the exponential feeding rate. $\mu$= specific bacterial growth rate [h$^{-1}$]; $X_0$= biomass at the beginning of the feeding [g/L]; t= time required for doubling [h]; $C_{Feed}$= concentration of glucose [g/L]; $Y_{X/S}$= Biomass yield [g/g]; $V_0$= starting volume [L]; F= feeding rate [mL/h].**
Before the feeding profile could be programmed (using the MFCS-software from B. Braun Biotec International / Satorius), a calibration of the feeding pump was performed. The feeding was performed at a $OD_{600}$-value of about 30. The time period of the feeding was 16 hours.

**Example 11 - Reference fermentations**

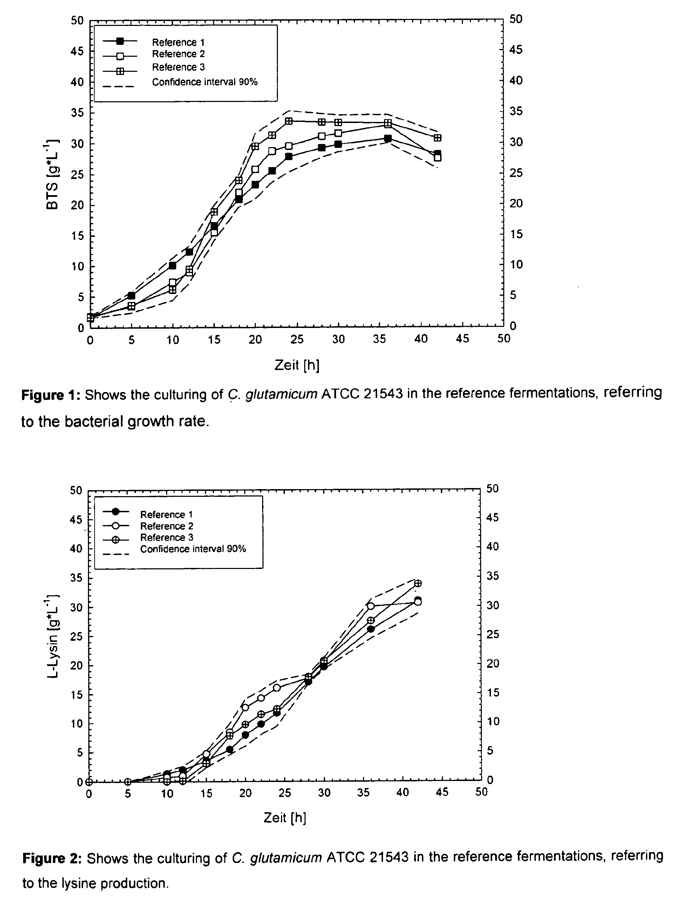

3 fermentation reactions without the vitamin compositions where performed as a reference. These fermentation reactions displayed similar characteristics referring to their values for the dry biomass formation. Figure 1 shows 4 phases of the culturing. The lag phase was between 0 and 5 h. After the lag phase, an exponential phase was observed, which lasted until 15 h. Subsequently, a transition phase started, which lasted until 25 h. The latter phase results from starting the feeding before threonine-limitation was observed. The feeding was started after 11 h of fermentation at a $OD_{600}$ of 30 and lasted for 16 h. Therefore, no quasi-steady state was observed in the fed-batch phase. After the transition phase ended, the culture remained in a stationary phase. The total operational time of the fermentation process was 42 h, which ended with depletion of the C-source.

The average bacterial growth rate of the reference-fermentations in the exponential phase was 0.13 h$^{-1}$ and the average final value for the dry biomass concentration (determined after 36 h) was 33 g L$^{-1}$. The overall biomass growth yield coefficient of the fermentation was 0.2 g g$^{-1}$.

The lysine production (Figure 2) began 5-10 h after starting the fermentation process. The culture was in the exponential phase und the threonine concentration in the medium was more than 200 mg/L at this point of time. The production of lysine continued until the end of the fermentation process in a linear manner and resulted in an average final concentration of 33 g L$^{-1}$. Accordingly, the overall average yield coefficient for lysine of the fermentation was 0.2 g g$^{-1}$, wherein the yield coefficient had its maximum value after 24-26 h of fermentation. The average glucose concentration of all reference fermentations reached its maximum after 26 h and an average concentration of 85 [g L$^{-1}$]. After this, the glucose concentration experienced a fast decline until the end of the fermentation process.

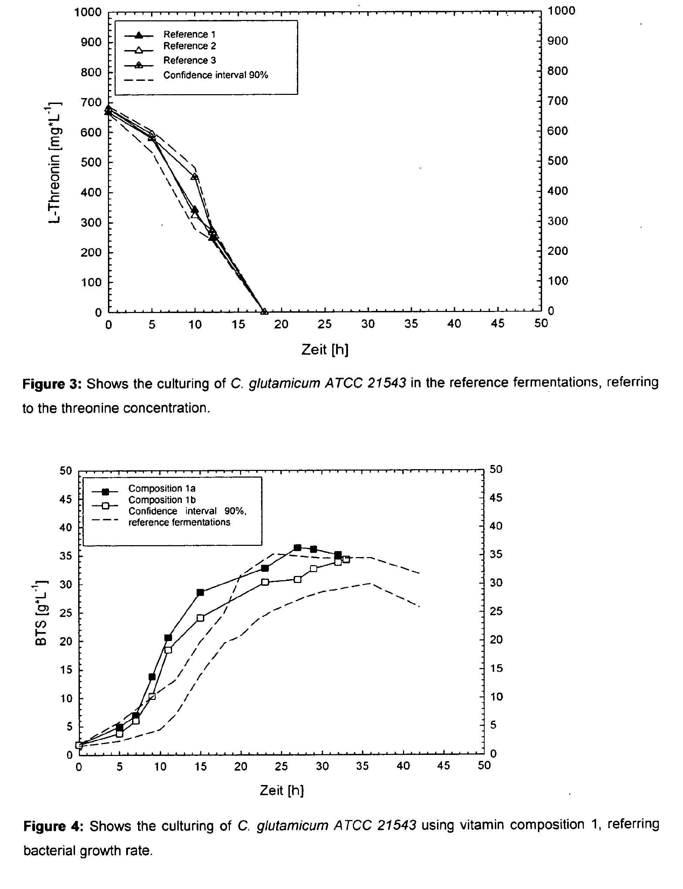

With the beginning of the growth, the threonine concentration (Figure 3) declined very fast and could not be determined after 18 h.

**Example 12 - Vitamin Composition 1**

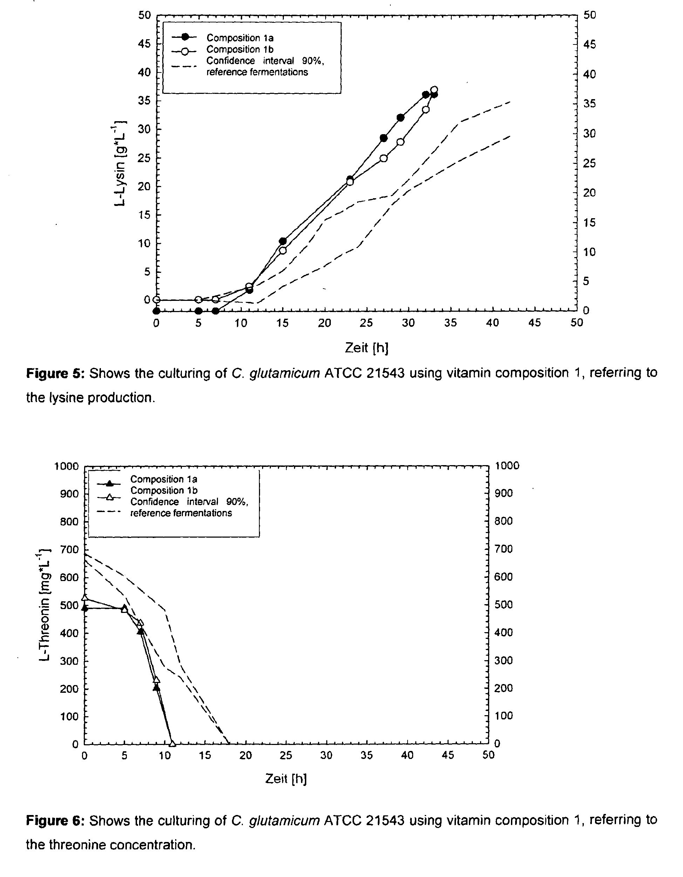

As can be derived from Figure 4, the lag phase was significantly shorter in the fermentation process, when using the vitamin composition 1. The exponential phase was also 5 h shorter and lasted only 6 h. Furthermore, the specific bacterial growth rate in the exponential phase was twice as high compared to the reference fermentations (average value of 0.26 h$^{-1}$). The exponential phase as a total was outside the confidence interval for the dry biomass concentration of the reference fermentations.

Without being obvious, the transition phase turns into the stationary phase, as the culture grows until the end of the fermentation. The overall operational fermentation time was 33 h, the average final dry biomass concentration of the fermentation with composition 1 was 34 g L$^{-1}$. Accordingly, the overall yield coefficient for the biomass was 0.2 g g$^{-1}$. The feeding lasted 16 h and was started after 7 h of fermentation at an $OD_{600}$ of 30 as well and ended after 23 h.

The production of lysine started - like in the reference fermentations - after 5-10 h. However, when using the vitamin composition 1 compared to the reference fermentations, the concentration increased much faster and ranged outside the confidence interval (Figure 5). The lysine production lasted until the end of the fermentation and reached an average concentration of 37 g L$^{-1}$. Accordingly, overall yield coefficient was 0.22 g g$^{-1}$. The overall yield coefficient for lysine, starting from the end of feeding until the end of the fermentation, was 0.28 g g$^{-1}$.

Compared to the reference fermentations, threonine was depleted after 11 h (5-7 h earlier), compare Figure 6. This is caused by the significantly increased growth from the begin of the fermentation on.

The glucose concentration reached its maximum of 55 g L$^{-1}$ after 23 h of fermentation. The significantly increased bacterial growth rate results in an increased cell number in this fermentation phase. Accordingly, more glucose is consumed at the beginning of the phase, which resulted in a depletion of the glucose level below 30 g L$^{-1}$ at this point of the fermentation. Compared to the reference fermentation, a significantly reduced maximum value for the glucose concentration was caused by that.

**Example 13 - Vitamin Composition 2**

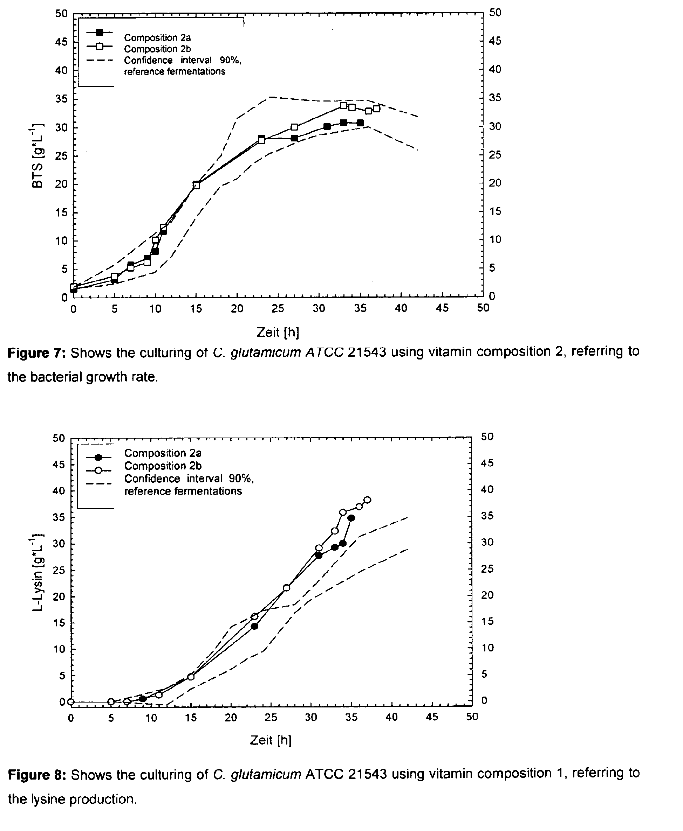

When using the vitamin composition 2, analog to the reference fermentations, a lag phase, an exponential phase, a transition phase and a stationary phase was observed (Figure 7). The exponential phase started after 5 h and lasted for 6 h.

The stationary phase of the fermentation with the composition 2 started after 33 h (about 25 h for the reference fermentations). The bacterial growths rate was 0.20 h$^{-1}$ and the final dry biomass concentration was 34 g L$^{-1}$ (also the maximum value). The average yield coefficient was 0.2 g g$^{-1}$.

The lysine concentration (Figure 8) started in the time interval of 5 h to 11 h of the fermentation and reached a concentration of 38 g L$^{-1}$ after 37 h. This resulted in an average product yield of 0.23 g g$^{-1}$. A maximum value of 76 g L$^{-1}$ was reached for the glucose concentration.

The lysine production crossed the confidence interval of the reference fermentations after about 25 h. The lysine production started earlier and displayed an enhanced increase compared to the reference fermentations.

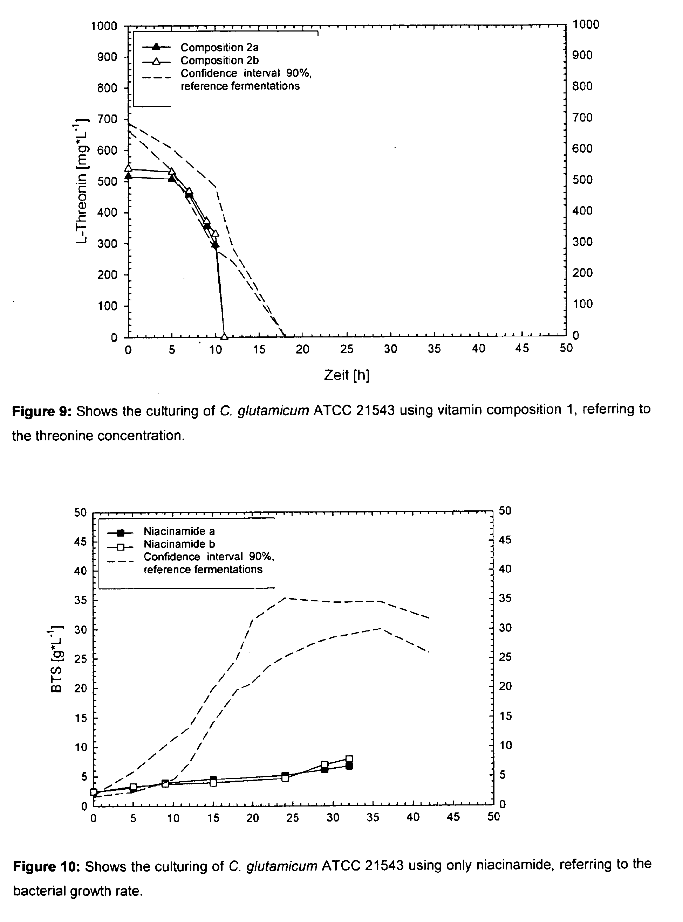

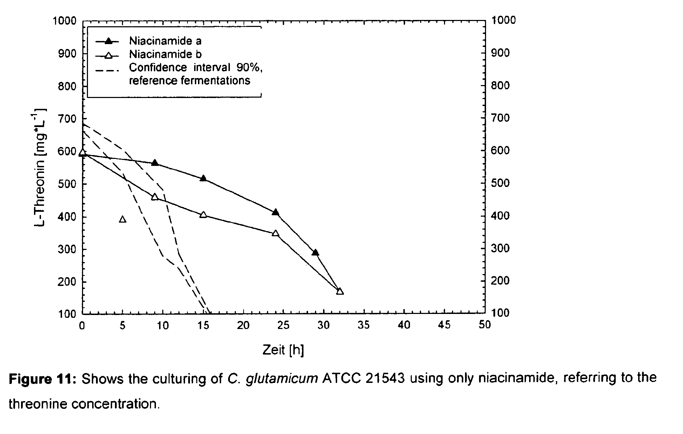

After 11 h of fermentation, threonine was completely depleted and the lysine production started (Figure 9). The average glucose concentration reached its maximum value at the end of the feeding (analog to the fermentation with vitamin composition 1, as well as the reference fermentations), which was 76 g L$^{-1}$.

**Example 14 - Niacinamide fermentation**

When performing fermentation processes using only niacinamide, no phase of a typical bacterial culture was observed. Therefore, the fermentation was stopped after 32 h. At the end of this fermentation process, a value of 9 g $L^{-1}$ was determined for the dry biomass concentration.

No feeding was performed, as the required $OD_{600}$ of 30 was not reached. As 12 g $L^{-1}$ of glucose was consumed, a yield of 0.5 g $g^{-1}$ was calculated. The threonine concentration (Figure 10) declined over the whole fermentation process, however, the decline was slower compared to the reference fermentation processes.

Over the whole fermentation process, no product formation could be observed. Contrary to that, an average threonine concentration of 300 mg $L^{-1}$ and a dry biomass concentration of 9 g $L^{-1}$ and 1 g $L^{-1}$ of lysine was observed in the previous fermentation processes.

A comparison of the results obtained in the different fermentation processes is given in Table 8.

**Table 8: Results of the fermentation processes.**

|  | Reference fermentations | Vitamin composition 1 | Vitamin composition 2 |
|---|---|---|---|
| Bacterial growth rate $\mu$ [$h^{-1}$] | 0,13 | 0,26 | 0,2 |
| Operational time of fermentation process [h] | 42 | 33 | 36 |
| max. dry biomass concentration [g $L^{-1}$] | 33 | 34 | 34 |
| max. lysine concentration [g $L^{-1}$] | 33 | 37 | 38 |
| Biomass yield, $Y_{X/S}$ [g $g^{-1}$] | 0,2 | 0,2 | 0,2 |
| Product yield, $Y_{P/S}$ in [g $g^{-1}$] | 0,2 | 0,22 | 0,23 |

## Claims

1. Use of vitamins in a fermentation process, **characterized in that** at least 4 vitamins selected from the group consisting of: thiamine, cobalamine, riboflavine, niacinamide, pantothenic acid, biotin, ascorbinic acid, retinol, procalciol, tocopherol, folic acid and pyridoxamine are used, and wherein the use of these vitamins enhances the bacterial growth rate ($\mu$) at least by 50 % and the average overall product concentration [g/L] at least by 10 % compared to a reference fermentation process, performed without adding vitamins.

2. Use of vitamins according to claim 1, **characterized in that** at least 5 vitamins selected from the group consisting of: thiamine, riboflavine, niacinamide, pantothenic acid, biotin and pyridoxamine, are used in the fermentation process.

3. Use of vitamins according to any of the preceding claims, **characterized in that** the vitamins are thiamine, riboflavine, nicacinamide, pantothenic acid, biotin and cobalamine.

4. Use of vitamins according to claim 1, **characterized in that** the products obtained in the fermentation process are selected from the group consisting of: amino acids, ethanol, citric acid, lactic acid, antibiotics, steroids, therapeutics and enzymes.

5. Use of a composition according to any of the preceding claims, **characterized in that** the microorganism for the fermentation process is *Corynebacterium glutamicum.*

6. Use of a composition according to any of the preceding claims, **characterized in that** the amino acid lysine is produced in the fermentation process.

7. Use of vitamins according to any of the preceding claims, **characterized in that** the vitamins reduce the duration of the lag phase at least by 15 % compared to a reference fermentation process, performed without adding vitamins.

8. Use of vitamins according to any of the preceding claims, **characterized in that** the use of the vitamins results in a reduction of the operational time of the fermentation process by at least 15 % compared to a reference fermentation process, performed without adding vitamins.

9. Use of vitamins according to any of the preceding claims, **characterized in that** the end concentration of all the vitamins, which are added in pure form is at least 19 mg $L^{-1}$ in the fermentation medium.

**Figure 1:** Shows the culturing of C. glutamicum ATCC 21543 in the reference fermentations, referring to the bacterial growth rate.

**Figure 2:** Shows the culturing of C. glutamicum ATCC 21543 in the reference fermentations, referring to the lysine production.

**Figure 3:** Shows the culturing of *C. glutamicum ATCC 21543* in the reference fermentations, referring to the threonine concentration.

**Figure 4:** Shows the culturing of *C. glutamicum ATCC 21543* using vitamin composition 1, referring bacterial growth rate.

**Figure 5:** Shows the culturing of *C. glutamicum* ATCC 21543 using vitamin composition 1, referring to the lysine production.

**Figure 6:** Shows the culturing of *C. glutamicum* ATCC 21543 using vitamin composition 1, referring to the threonine concentration.

**Figure 7:** Shows the culturing of *C. glutamicum ATCC* 21543 using vitamin composition 2, referring to the bacterial growth rate.

**Figure 8:** Shows the culturing of *C. glutamicum* ATCC 21543 using vitamin composition 1, referring to the lysine production.

**Figure 9:** Shows the culturing of *C. glutamicum* ATCC 21543 using vitamin composition 1, referring to the threonine concentration.

**Figure 10:** Shows the culturing of *C. glutamicum* ATCC 21543 using only niacinamide, referring to the bacterial growth rate.

**Figure 11:** Shows the culturing of *C. glutamicum* ATCC 21543 using only niacinamide, referring to the threonine concentration.

**European Patent Office**

**PARTIAL EUROPEAN SEARCH REPORT**

which under Rule 45 of the European Patent Convention shall be considered, for the purposes of subsequent proceedings, as the European search report

**Application Number**

EP 06 02 2431

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GB 1 430 842 A (AJINOMOTO KK) 7 April 1976 (1976-04-07) * page 1, line 31 - page 6, line 10; tables 1,2 * | 1-9 | INV. C12P13/08 |
| Y | WO 03/012058 A (UNIV NORTH CAROLINA [US]; BREITSCHWERDT EDWARD B [US]; SONTAKKE SUSHAM) 13 February 2003 (2003-02-13) * page 7, line 32 - page 8, line 8 * * page 32, line 14 - page 35, line 8 * | 1-9 | |
| A | DATABASE WPI Week 197502 Derwent Publications Ltd., London, GB; AN 1975-02617W XP002428801 & JP 49 066890 A (HITACHI CHEM IND CO) 28 June 1974 (1974-06-28) * abstract * | 1-9 | |
| A | GB 1 449 087 A (DJINOMOTO CO INC) 8 September 1976 (1976-09-08) * the whole document * | 1 | |

TECHNICAL FIELDS SEARCHED (IPC)

C12P

-/--

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Berlin | 17 April 2007 | De Kok, Ad |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent**    **PARTIAL EUROPEAN SEARCH REPORT**    Application Number

**Office**

EP 06 02 2431

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| D,A | KISS R D ET AL: "METABOLIC ACTIVITY CONTROL OF THE L LYSINE FERMENTATION BY RESTRAINED GROWTH FED-BATCH STRATEGIES" BIOTECHNOLOGY PROGRESS, vol. 7, no. 6, 1991, pages 501-509, XP002428743 ISSN: 8756-7938 * page 501 - page 502 * ----- | 1-9 | |

TECHNICAL FIELDS SEARCHED    (IPC)

EPO FORM 1503 03.82 (P04C10)

| | | Application Number |
|---|---|---|
| **European Patent Office** | **INCOMPLETE SEARCH SHEET C** | **EP 06 02 2431** |

Claim(s) searched completely:
    2-9

Claim(s) searched incompletely:
    1

Reason for the limitation of the search:

Present claim 1 relates to a use which has a given desired property or effect, namely to enhance bacterial growth in a fermentation process. The claim encompasses any fermentation process for any product and any microorganism whereas the description provides support and disclosure in the sense of Article 83 and 84 EPC only for lysine as fermentation product and only for Corynebacterium glutamicum  as microorganism.  This non-compliance with the substantive provisions is to such an extent, that a meaningful search of the whole claimed subject-matter of the claim could not be carried out (Rule 45 EPC and Guidelines B-VIII, 3).

The search of claim 1 was consequently restricted to the subject-matter of claims 5 and 6.

## ANNEX TO THE EUROPEAN SEARCH REPORT
## ON EUROPEAN PATENT APPLICATION NO.

EP 06 02 2431

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2007

| Patent document cited in search report | | Publication date | Patent family member(s) | | Publication date |
|---|---|---|---|---|---|
| GB 1430842 | A | 07-04-1976 | AU | 5463773 A | 24-10-1974 |
| | | | BG | 24960 A3 | 15-06-1978 |
| | | | CH | 574501 A5 | 15-04-1976 |
| | | | CS | 182785 B2 | 31-05-1978 |
| | | | DE | 2321461 A1 | 08-11-1973 |
| | | | DK | 134570 B | 29-11-1976 |
| | | | FR | 2182211 A1 | 07-12-1973 |
| | | | HU | 172604 B | 28-11-1977 |
| | | | NL | 7305828 A | 30-10-1973 |
| | | | SE | 410978 B | 19-11-1979 |
| | | | SU | 526295 A3 | 25-08-1976 |
| WO 03012058 | A | 13-02-2003 | AU | 2002332438 A1 | 17-02-2003 |
| JP 49066890 | A | 28-06-1974 | NONE | | |
| GB 1449087 | A | 08-09-1976 | AR | 200781 A1 | 13-12-1974 |
| | | | FR | 2230723 A1 | 20-12-1974 |
| | | | IT | 1012768 B | 10-03-1977 |
| | | | US | 3929571 A | 30-12-1975 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NAKAYAMA et al.** *J. Gen. Appl. Microbiol.,* 1961, vol. 7, 145-154
- **TAKINAMI et al.** *Biol. Chem.,* 1965, vol. 29, 351-359
- **KISS et al.** *Biotechnol. Prog.,* 1991, vol. 7, 501-508
- **EINARSSON et al.** *Anal. The.,* 1986, vol. 58, 1638-1643